Europäisches Patentamt

European Patent Office

Office européen des brevets

(11) Publication number: **0 412 155 A1**

## (12) EUROPEAN PATENT APPLICATION
### published in accordance with Art. 158(3) EPC

(21) Application number: **88902926.0**

(22) Date of filing: **25.03.88**

(86) International application number:
**PCT/JP88/00308**

(87) International publication number:
**WO 88/07676 (06.10.88 88/22)**

(51) Int. Cl.⁵: **G01N 27/30, G01N 27/28**

(30) Priority: **27.03.87 JP 71830/87**
**27.03.87 JP 71831/87**

(43) Date of publication of application:
**13.02.91 Bulletin 91/07**

(84) Designated Contracting States:
**BE DE FR GB IT NL SE**

(71) Applicant: **TERUMO KABUSHIKI KAISHA**
**No. 44-1, Hatagaya 2-chome, Shibuya-ku**

**Tokyo 151(JP)**

(72) Inventor: **KATSUBE, Teruaki**
**1-1-205, 3-chome Nagayama**
**Tama-shi Tokyo 206(JP)**

(74) Representative: **Gillard, Marie-Louise et al**
**Cabinet Beau de Loménie 55, Rue**
**d'Amsterdam**
**F-75008 Paris(FR)**

(54) **ENZYME SENSOR.**

㊲ According to the present invention, an enzyme sensor having an immobilized enzyme film is preserved while being soaked in a solution having a predetermined pH or kept in an atmosphere not containing any heavy metal. The enzyme sensor thus preserved has less degradation resulting from deactivation of enzyme and can provide a stable measurement for a long period.

*FIG.4*

## ENZYME SENSOR

### TECHNICAL FIELD

This invention relates to an enzyme sensor and, more particularly, to an enzyme sensor in which a fixed-enzyme film is formed.

### BACKGROUND ART

Moriizumi et. al. have reported [Nihon Kagaku Kaishi, No. 6, (1983)] on a glass electrode-type ion-sensitive electrode, a miniature tip-type ion-sensitive electrode utilizing semiconductor machining technology, and an enzyme sensor in which an enzyme-fixed film is formed on an ion-sensitive film of an ion-sensitive field-effect transistor (ISFET) utilizing semiconductor device technology.

An ion-sensitive film is one in which the surface potential of a film varies in dependence upon the concentration of an ion in solution. By measuring this potential, the ion concentration of the solution can be determined. By fixing an enzyme film, which is accompanied by an increase or decrease in hydrogen ion due to an enzyme reaction, on this ion-sensitive film, an enzyme sensor is formed which makes possible measurement in a continuous and simple manner.

Since the lifetime of this enzyme sensor is short, however, stable measurement over a long period of time cannot be carried out. In particular, when an enzyme sensor is preserved in a solution, any heavy metal (e.g., Mn) ions in the solution react with the enzyme and cause it to deactivate, as a consequence of which the lifetime of the enzyme sensor is shortened to make long-term, stable measurement impossible.

### DISCLOSURE OF THE INVENTION

The present invention provides an enzyme sensor in which deterioration of the sensor is prevented to make possible long-term, stable measurement. As means for solving the foregoing problem, the enzyme sensor of the present invention has a fixed-enzyme film and is immersed in a solution having a predetermined pH. In such an arrangement, deterioration of the fixed-enzyme film is prevented by the predetermined pH solution, so that there can be provided an enzyme sensor which is prevented from deteriorating, thereby enabling stable measurement over a long period of time.

Preferably, the predetermined pH solution is a buffer solution, and the predetermined solution contains ethylenediamine tetraacedic acid (EDTA).

As other means for solving the foregoing problem, the enzyme sensor of the present invention has a fixed-enzyme film and is present in an atmosphere that does not contain a heavy metal. In such an arrangement, deterioration of the fixed-enzyme film due to heavy metals is prevented and deactivation of the enzyme caused by heavy metals is prevented in the heavy metal-free atmosphere. Thus there can be provided an enzyme sensor which is prevented from deteriorating, thereby making possible long-term, stable measurement.

Preferably, the heavy metal-free atmosphere is formed from dry air and a member enclosing the dried air. Further, the heavy metal-free atmosphere is formed from a prescribed gas and a member enclosing the prescribed gas.

### BRIEF DESCRIPTION OF THE DRAWINGS

Fig. 1 is a view illustrating a change in lifetime of a urease film enzyme sensor when the type of buffer solution in which the sensor is preserved is changed;

Fig. 2 is a view illustrating a change in lifetime of a urease film enzyme sensor depending upon the type of buffer solution when EDTA is incorporated in the buffer solution preserving the sensor;

Fig. 3 is a view illustrating a change in lifetime of a urease film enzyme sensor when preservation is carried out in dry air at a temperature of 4°C and the buffer solution of measurement is changed; and

Figs. 4(a) - (c) are views showing examples of enzyme sensors.

### BEST MODE FOR CARRYING OUT THE INVENTION

(1) Preparation of pH sensor

A pH sensor can be prepared by forming a film of iridium oxide ($IrO_x$), which is a hydrogen ion-sensitive film, on ITO glass, a quartz substrate or a sapphire substrate. It is also possible to prepare a pH sensor by forming iridium oxide ($IrO_x$) as the sensitive film of an ISFET on the gate of a FET or on a substrate extending from a gate.

This sensitive film of iridium oxide ($IrO_x$) was formed by reactive sputtering using pure oxygen, with iridium serving as a target. Pressure within a bell jar at the time of sputtering was about $10^{-3}$

torr (= mmHg), and the thickness of the iridium oxide (IrO$_x$) film formed was about 800 Å. In order to obtain a pH sensor output, lead wires were brought into contact with the sensor by an electrically conductive adhesive after formation of the iridium oxide (IrO$_x$) film. In order to protect the electrode portions, the pH sensor was molded in epoxy resin or silicone resin.

(2) Preparation of enzyme sensor

An enzyme sensor is fabricated by forming a fixed-enzyme film on the ion-sensitive film of the pH sensor prepared in accordance with method (1). The method of forming the fixed-enzyme film relies upon cross-linking. Dissolved in a 0.1 M, pH 7.5 HEPES (N-2-hydroxyethylpiperazine-N-2-ethanesulfonic acid) buffer solution were 300 mg/ml of cow serum albumin and 50 mg/ml of urease. Glutaric aldehyde was mixed with this solution, the resulting mixture was dropped onto the iridium oxide (IrO$_x$) film of the pH sensor, and the sensor was immediately rotated at about 3000 rpm for one minute (preparation by the spin coating method). As a result, it was possible to obtain an enzyme sensor which, in the present embodiment, is a urea sensor, having a highly uniform, thin (about 1 $\mu$m) fixed-enzyme film.

(3) Preservation of enzyme sensor The enzyme sensor fabricated in accordance with (2) above was measured at a fixed urea concentration of 100 mg/dl in a HEPES - NaOH buffer solution, a phosphate buffer solution and in a tris-HCl buffer solution (all of which were 0.1 M, pH 7.5 buffer solutions at a temperature of 30°C). After the output voltage of the enzyme sensor was measured using Ag/AgCl as a reference electrode, the enzyme sensor was immersed and preserved in various solutions and the state of deterioration was tested, as indicated in each of the following Examples. The pH of the solution preferably is in a range which will not deactivate the enzyme. A range of 5.0 - 8.0 is preferred, especially a range of 7.4 - 8.0. A preferred temperature range is 20°C - -20°C, which will not result in deactivation of the enzyme. A range of 4°C - 0°C is especially preferred.

<Example 1>

The enzyme sensor was immersed in a 0.1 M, pH 7.5 tris-HCL buffer solution at a temperature of 4°C and measured in a 10 mM tris-HCl buffer solution (thickness of fixed-enzyme film: 1 $\mu$m).

<Example 2>

The enzyme sensor was immersed in a 0.1 M, pH 7.5 phosphate buffer solution at a temperature of 4°C and measured in a 25 mM tris-HCl buffer solution (thickness of fixed-enzyme film: 1 $\mu$m).

<Example 3>

The enzyme sensor was immersed in a 0.1 M, pH 7.5 HEPES-NaOH buffer solution at a temperature of 20°C and measured in a 20 mM KEPES-NaOH buffer solution (thickness of fixed-enzyme film: 1 $\mu$m).

<Example 4>

The enzyme sensor was immersed in a tris-HCL buffer solution containing 1 mM EDTA (ethylenediamine tetraacedic acid (thickness of fixed-enzyme film: 10 um).

<Example 5>

The enzyme sensor was immersed in a phosphate buffer solution containing 1 mM EDTA (ethylenediamine tetraacedic acid (thickness of fixed-enzyme film: 10 um).

<Example 6>

The enzyme sensor was immersed in a HEPES-NaOH buffer solution containing 1 mM EDTA (ethylenediamine tetraacedic acid (thickness of fixed-enzyme film: 10 um).

<Results of Examples 1 - 3>

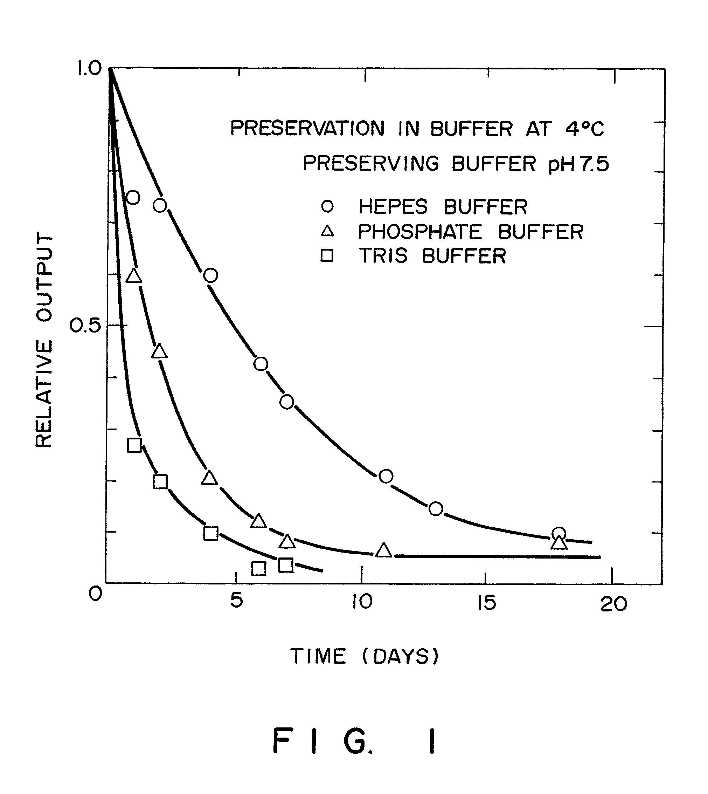

Fig. 1 shows changes, with the passage of time, in the output of the enzyme sensor fabricated in accordance with the present embodiment, the differences in the curves being attributable to the different buffer solutions used in immersion and measurement. It will be understood that the rate of deterioration differs depending upon the buffer solution, wherein the use of the phosphate solution somewhat suppresses deterioration in comparison with use of the tris-HCl buffer solution, with deterioration being reduced to an even greater degree by using the HEPES-NaOH buffer solution.

<Results of Examples 4 - 6>

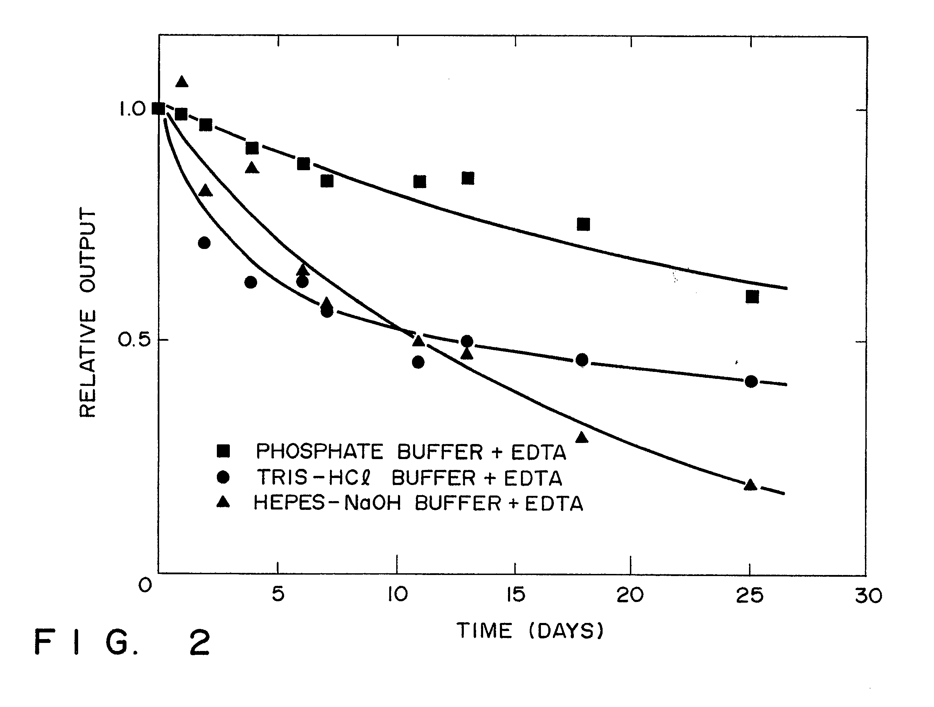

Fig. 2 shows changes, with the passage of time, in the output of the enzyme sensor fabricated in accordance with the present embodiment, based on different buffer solutions when the sensor is preserved in buffer solutions containing 1 mM EDTA. It will be understood that incorporating the EDTA in the buffer solutions. suppresses deterioration in all cases in comparison with the results shown in Fig. 1. It will be appreciated that when EDTA is incorporated in the phosphate buffer solution, deterioration can be suppressed to a major degree. Here the buffer solutions refer to solutions the pH whereof is held constant so as not to deactivate the enzyme.

After the output voltage of an enzyme sensor similar to the foregoing was measured, the state of deterioration of the sensor was tested in the environments indicated in each of the following examples.

<Example 7>

The sensor was preserved in air at 4°C, and the measurement buffer solution was tris-HCl (thickness of fixed-enzyme film: 10 $\mu$m).

<Example 8>

The sensor was preserved in air at 4°C, and the measurement buffer solution was phosphate (thickness of fixed-enzyme film: 10 $\mu$m).

<Example 9>

The sensor was preserved in air at 4°C, and the measurement buffer solution was HEPES-NaOH (thickness of fixed-enzyme film: 10 $\mu$m).

<Results of Examples 7 - 9>

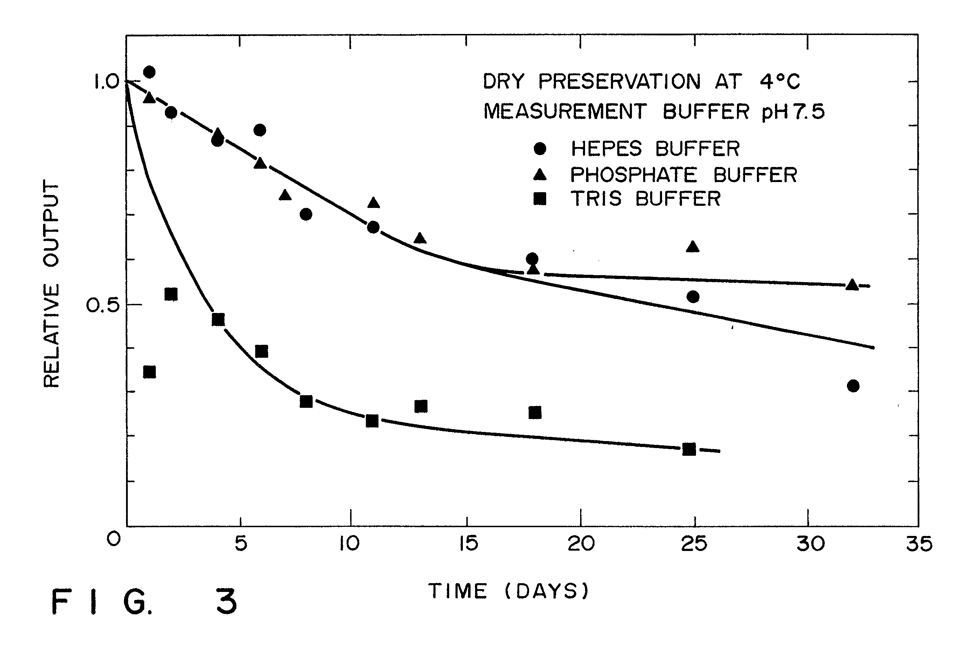

Fig. 3 shows changes, with the passage of time, in the output of the enzyme sensor fabricated in accordance with the present embodiment when the sensor is preserved in air at 4°C and the measurement buffer solutions are changed. As before, deterioration differs depending upon the type of buffer solution. In comparison with the tris-HCl buffer solution, deterioration is smaller using the phosphate buffer solution or the HEPES-NaOH buffer solution. Preferably, the pH range of the buffer solutions is one which will not result in deactivation of the enzyme. An excellent pH range is 5.0 - 8.0, with 7.4 - 8.0 being especially preferred. As for temperature, a range of 20° - -20°C is preferred since this will not cause deactivation of the enzyme. A particularly preferred range is 4°C - 0°C. Here the buffer solutions refer to solutions the pH whereof is held constant so as not to deactivate the enzyme. It is self-evident that an atmosphere substantially free of heavy metals will produce the same effects as an $O_2$ gas atmosphere, an $N_2$ gas atmosphere and an atmosphere of inert gas such as Ar.

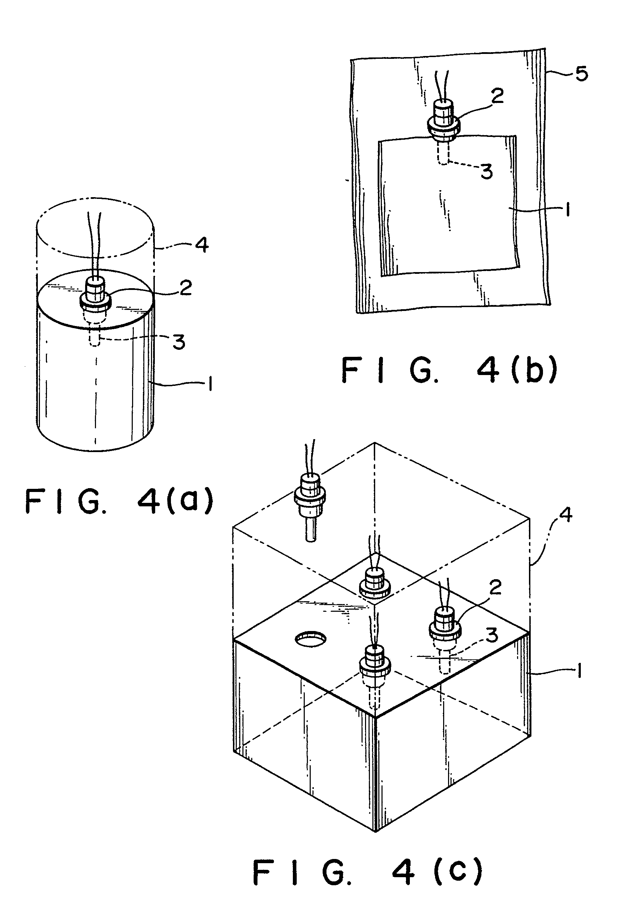

Figs. 4(a) - (c) illustrate examples of enzyme sensors that can be contrived based on the results of Examples 1 - 9. Numeral 1 denotes an enclosure, 2 the main body of an enzyme sensor, 3 an enzyme-sensitive portion having a fixed-enzyme film, 4 a cover and 5 a sheath. The interior of the enclosure 1 is filled with a buffer solution, a buffer solution containing EDTA, dry air or any of the abovementioned gases.

Examples of materials that can be used for the enclosure 1 include vinyl chloride, AS resin, polycarbonate, polystyrene, polyolefin (especially propylene and polyethylene), polyethylene chloride, ethylene-vinyl acetate copolymer (EVA) and glass.

Though the enzyme sensor in the present embodiment is typified by a urea sensor, it is self-evident that the same results will be obtained even with enzyme sensors having other fixed-enzyme films.

## Claims

1. An enzyme sensor having a fixed-enzyme film, characterized in that said enzyme sensor is immersed in a prescribed pH solution.

2. An enzyme sensor according to claim 1, characterized in that the prescribed pH solution is a buffer solution.

3. An enzyme sensor according to claim 2, characterized in that prescribed pH solution contains ethylenediamine tetraacedic acid (EDTA).

4. An enzyme sensor having a fixed-enzyme film, characterized in that said enzyme sensor is present in an atmosphere that is free of heavy metals.

5. An enzyme sensor according to claim 4, characterized in that the atmosphere that is free of heavy metals is formed from dry air and a member enclosing said dry air.

6. An enzyme sensor according to claim 4, characterized in that the atmosphere that is free of heavy metals is formed from a prescribed gas and a member enclosing said prescribed gas.

PRESERVATION IN BUFFER AT 4°C

PRESERVING BUFFER pH 7.5

○ HEPES BUFFER
△ PHOSPHATE BUFFER
□ TRIS BUFFER

F I G. I

FIG. 2

EP 0 412 155 A1

DRY PRESERVATION AT 4°C
MEASUREMENT BUFFER pH 7.5

● HEPES BUFFER
▲ PHOSPHATE BUFFER
■ TRIS BUFFER

RELATIVE OUTPUT

TIME (DAYS)

FIG. 3

EP 0 412 155 A1

F I G.  4(a)

F I G.  4(b)

F I G.  4(c)

# INTERNATIONAL SEARCH REPORT

International Application No PCT/JP88/00308

**I. CLASSIFICATION OF SUBJECT MATTER** (if several classification symbols apply, indicate all)

According to International Patent Classification (IPC) or to both National Classification and IPC

Int.Cl$^4$    G01N27/30, G01N27/28

**II. FIELDS SEARCHED**

Minimum Documentation Searched

| Classification System | Classification Symbols |
|---|---|
| IPC | G01N27/28, 27/30, 27/46-56 |

Documentation Searched other than Minimum Documentation
to the Extent that such Documents are Included in the Fields Searched

| | |
|---|---|
| Jitsuyo Shinan Koho | 1926 - 1988 |
| Kokai Jitsuyo Shinan Koho | 1971 - 1988 |

**III. DOCUMENTS CONSIDERED TO BE RELEVANT**

| Category* | Citation of Document, with indication, where appropriate, of the relevant passages | Relevant to Claim No. |
|---|---|---|
| X | JP, A, 62-32353 (Fuji Electric Co., Ltd.) 12 February 1987 (12. 02. 87) (Family: none) | 1-4 |
| X | JP, A, 59-58355 (Hitachi, Ltd.) 4 April 1984 (04. 04. 84) (Family: none) | 1-4 |
| X | Bunseki, 1977 No. 11, (November 1977) (Tokyo) Kawashima Takuji, "Koso Denkyoku", P.681-692 Especially p.684, left column, 13th line from the bottom to 8 | 1-6 |

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| June 6, 1988 (06. 06. 88) | June 27, 1988 (27. 06. 88) |

| International Searching Authority | Signature of Authorized Officer |
|---|---|
| Japanese Patent Office | |

Form PCT ISA 210 (second sheet) (January 1985)